# EUROPÄISCHE PATENTANMELDUNG

(11) **EP 3 628 255 A1**
(43) Veröffentlichungstag der Anmeldung: **01.04.2020**
(21) Anmeldenummer: 18196945.2
(22) Anmeldetag: 26.09.2018
(51) Int. Cl.: A61B 18/14

(54) **HF-CHIRURGISCHES PRÄPARATIONSINSTRUMENT MIT FLUIDKANAL**

(71) Anmelder: Erbe Elektromedizin GmbH, 72072 Tübingen (DE)
(72) Erfinder: FISCHER, Klaus, 72202 Nagold (DE); ENDERLE, Markus, 72070 Tübingen (DE); BRODBECK, Achim, 72555 Metzingen (DE); NEAGOS, Alexander, 72770 Reutlingen (DE); FECH, Andreas, 72072 Tübingen (DE)
(74) Vertreter: Rüger Abel Patentanwälte PartGmbB

(57) **Zusammenfassung**

Es wird ein Präparationsinstrument (10) angegeben, welches ein HF-Instrument mit einer mittels eines Isolierkörpers (16) teilweise isolierten Elektrode (13) aufweist, welches mit einem Fluidapplikator mit einem in dem Isolierkörper (16) angeordneten Kanal (35) zur Applikation eines Fluids an oder in Gewebe kombiniert ist. In besonders bevorzugten Ausführungsformen des Präparationsinstruments (10) ist die Elektrode (13) eine Spatelelektrode, welche in dem Isolierkörper (16) steckt, welcher Abschnitte (24, 25a, 25b, 63, 64) der Oberfläche der Elektrode (13) nicht bedeckt, so dass diese Abschnitte (24, 25a, 25b, 63, 64) Gewebekontakt haben können. In den besonders bevorzugten Ausführungsformen bildet der Isolierkörper (10) vorzugsweise die den Kanal (35) begrenzende Kanalwand. In den besonders bevorzugten Ausführungsformen sind der Isolierkörper (16) und die Elektrode (13) flexibel, um die Form des Isolierkörpers (16) und der Elektrode (13) zusammen an die chirurgische Aufgabe anzupassen.

## Beschreibung

Die Erfindung betrifft ein HF-chirurgisches Präparationsinstrument.

Neben dem rein mechanischen Präparieren mit Schere und Pinzette sind die diathermischen Verfahren (Hochfrequenz(HF)-chirurgische Verfahren) heute das Mittel der Wahl. Derzeit werden zur Präparation von Gewebestrukturen nicht-isolierte Spatel- oder Hakenelektroden verwendet. Teilweise isolierte Elektroden hingegen finden insbesondere dann ihre Anwendung, wenn eine thermische Schädigung der freizustellenden oder angrenzenden Strukturen möglichst gering sein soll. Bei bekannten Präparationsinstrumenten kann das präzise und sichere Arbeiten insbesondere durch eingeschränkte Sicht auf den OP-Situs, zum Beispiel in Folge von Sickerblutungen, karbonisiertem Blut und Gewebe sowie Überdeckungen der Instrumentenspitze (des aktiven Bereichs) durch "fließendes" Gewebe erschwert sein.

Aus US 2006/0 111 709 A1 ist ein elektrochirurgisches Instrument bekannt, welches eine Hakenelektrode aufweist, wobei Kühlfluid von einem Reservoir durch den Instrumentenkörper, an die Elektrodenspitze, durch eine Öffnung in der Elektrode und von dort in einen Rückführungskanal zum Reservoir geleitet werden kann. Dadurch ist die Elektrode kühlbar.

WO 95/19 739 A offenbaren ein elektrochirurgisches Instrument mit einer Elektrode mit einem freien Ende, wobei die Elektrode bis vor das Ende von einem Mantel aus elektrisch isolierendem Material umgeben ist, wobei in einem Abschnitt des Mantels, welcher an das Ende angrenzt, Öffnungen in dem Mantel enthalten sind, die von der Elektrode zu der Oberfläche des Mantels reichen, um Gewebe durch die Öffnungen mit HF-Leistung zu beaufschlagen.

WO 2016/0731 64 A1 zeigt ein elektrochirurgisches Instrument mit einer nicht isolierten Elektrode, die einen Kanal umschließt, durch welchen Gas aus einer Öffnung am Ende der Elektrode ausgestoßen wird, um Sauerstoff von dem Ende der Elektrode zu verdrängen.

Auf der Webseite http://mmcts.org/tutorial/830#additionalinfo wird ein Instrument offenbart, welches aus einem elektrochirurgischen Instrument und einem Instrument zur Ausbringung eines CO₂- und Kochsalzlösung-Gemisches zusammengesetzt ist, wobei das elektrochirurgische Instrument und das weitere Instrument mit Fäden verbunden sind.

Ausgehend von diesem Stand der Technik ist es Aufgabe der vorliegenden Erfindung, ein verbessertes Instrument bereitzustellen.

Diese Aufgabe wird mit einem HF-chirurgischen Präparationsinstrument nach Anspruch 1 sowie einer Vorrichtung nach Anspruch 15 gelöst:

Das HF-chirurgische Präparationsinstrument (im Folgenden auch Präparationsinstrument oder Instrument genannt) weist eine Elektrode zum Beaufschlagen von Gewebe mit HF-Leistung auf, wobei Oberflächenabschnitte der Elektrode mittels eines Isolierkörpers nach außen elektrisch und thermisch isoliert sind. Innerhalb des Isolierkörpers ist ein Kanal angeordnet, der zum Ausbringen eines Fluides, wie eines Gases, einer Flüssigkeit und/oder eines Aerosols, auf oder in das Gewebe oder zwischen Gewebestrukturen eingerichtet ist. Der Kanal ist an eine Fluidquelle, beispielsweise Gas-, Flüssigkeits- und/oder Aerosolquelle, anschließbar, insbesondere angeschlossen.

Das Instrument ist vorzugsweise ein monopolares HF-Instrument. Der HF-Generator, welcher das Instrument speist, ist mit einer gesonderten Neutralelektrode verbunden, welche den Patienten großflächig elektrisch kontaktiert.

Mit dem HF-chirurgischen Präparationsinstrument kann Gas, Flüssigkeit und/oder Aerosol in oder neben einen Einwirkungsbereich der HF-Leistung gebracht werden. Das Präparationsinstrument bildet entsprechend einen Fluidapplikator.

Unter einem Aerosol wird im Sinne dieser Anmeldung ein Gemisch aus in einem Gas und einer Flüssigkeit, insbesondere aus in einem Gas schwebenden Flüssigkeitströpfchen, verstanden.

Mit dem erfindungsgemäßen Instrument lässt sich Gewebe schonend und präzise präparieren. Unter Präparation wird hier die Freilegung und Darstellung von vulnerablen anatomischen Strukturen, wie z.B. Nerven oder Gefäßen durch stumpfes Auseinanderschieben/Abdrängen und/oder scharfes Trennen verstanden. Bei der HF-chirurgischen Präparation werden z.B. mit Hilfe eines HF-Spatels bestimmte Gewebestrukturen wie Gefäße, Nerven oder Organe zumindest teilweise aus dem Gewebeverbund herausgelöst. Bei diesem Schritt ist es entscheidend, dass es zu keiner unbeabsichtigten thermischen Schädigung sensibler (zu präparierender) oder angrenzender Strukturen kommt. Das erfindungsgemäße Instrument gewährleistet, dass eine Kontaktfläche zwischen dem Gewebe und der HF-Elektrode zu jedem Zeitpunkt bekannt bzw. kontrollierbar ist. Durch Ab- oder Verdrängen von Gewebe, Gewebestrukturen, Geweberesten, Körperflüssigkeiten oder sonstigen Flüssigkeiten oder Nebeln mittels der Gas-, Flüssigkeits-, und/oder Aerosolapplikatorfunktion des erfindungsgemäßen Instruments kann der Chirurg die einzelnen Gewebegrenzen stets deutlich erkennbar machen.

Der Kanal verläuft bevorzugt neben der Elektrode, mit oder ohne Abstand zu der Elektrode. Alternativ kann die Elektrode den Kanal enthalten. Bevorzugt jedoch begrenzt der Isolierkörper den Kanal. Dies bedeutet, dass der Isolierkörper zumindest abschnittsweise die Kanalwand bildet. In dem Abschnitt kann beispielsweise auf ein Röhrchen in dem Isolierkörper verzichtet werden, wobei das Röhrchen den Kanalabschnitt enthält. Der Kanal verläuft vorzugsweise innerhalb der Wandstärke des Isolierkörpers. Die Elektrode verläuft bevorzugt mit Abstand zu dem Kanal entlang des Kanals. Die Elektrode ist bevorzugt nicht in dem Kanal angeordnet und reicht bevorzugt nicht in den Kanal hinein, so dass der Kanal die Elektrode in einer Querschnittsebene durch den Kanal gesehen vorzugsweise nicht umgibt.

Bevorzugt weist der Kanal einen Düsenabschnitt als Endabschnitt auf, in welchem sich der Querschnitt, insbesondere Durchmesser des Kanals, verjüngt. Der Düsenabschnitt wird vorzugsweise von dem Isolierkörper begrenzt. Dies bedeutet, dass der Isolierkörper die Wand des Düsenabschnitts des Kanals bildet.

Der distale Endabschnitt (Instrumentenspitze), insbesondere die Elektrode und der Isolierkörper, ist vorzugsweise biegbar, um die Form des Isolierkörpers und der Elektrode an die Behandlungsaufgabe anzupassen. Wenn die äußere Kraft zum Verbiegen weggenommen wird, bleibt die gewünschte Form des Endabschnitts des Isolierkörpers vorzugsweise erhalten. Die Ausrichtung der Düse des Kanals relativ zu der Elektrodenspitze bleibt vorzugsweise erhalten. Vorzugsweise ist der Endabschnitt mit der Hand biegbar. Wenn der Endabschnitt in die gewünschte Form biegbar ist, fördert dies eine präzise und gewebeschonende Präparation, wie zum Beispiel bei der Gefäßentnahme.

Durch die Isolation kann die elektrische Kontaktfläche zwischen der Elektrode und dem Gewebe auf einen definierten und eng begrenzten Bereich festgelegt werden. Dadurch sinkt zum einen die in das Gewebe eingebrachte Energie. Zum anderen werden durch den begrenzten Wirkbereich der Lateralschaden sowie das Risiko einer unbeabsichtigten thermischen Beanspruchung des umliegenden Gewebes minimiert.

Wenn der Isolierkörper den Kanal begrenzt, insbesondere wenn der Isolierkörper den Endabschnitt des Kanals begrenzt, der an die Öffnung des Kanals zum Auslass des Aerosols oder der Flüssigkeit in das Einwirkgebiet des Instruments gestattet, führt dies dazu, dass bei Verbiegung des Instruments die Ausrichtung (Winkel) des Endabschnitts des Kanals relativ zu der Ausrichtung des distalen Endabschnitts der Elektrode erhalten bleibt.

Die Elektrode ist bevorzugt nicht in einem Kanal angeordnet, der zum Führen eines Fluidstroms bestimmt wäre. Die Elektrode kann in einer Ausnehmung in dem Isolierkörper angeordnet sein. Die Elektrode kann die Ausnehmung vollständig ausfüllen oder zumindest die Öffnung in die Ausnehmung verschließen.

In bevorzugten Ausführungsformen weist die Elektrode eine Spatelform auf. Dies bedeutet, dass die Elektrode zumindest einen spatelförmigen distalen Endabschnitt aufweist. Das Instrument bildet mit einer spalteförmigen Elektrode vorzugsweise einen HF-Spatel. In bevorzugten Ausführungsformen weist die Elektrode einen drahtförmigen Abschnitt auf, der sich vorzugsweise entlang der Längserstreckung der Elektrode erstreckt. Alternativ kann die Elektrode beispielsweise einen länglichen Schlitz oder Ausnehmung aufweisen, die sich beispielsweise vom proximalen Ende der Elektrode bis zum Endabschnitt der Elektrode erstrecken kann, wobei in dem Schlitz oder Ausnehmung ein drahtförmiger Körper, vorzugsweise Metallkörper, angeordnet ist. Der drahtförmige Körper ist vorzugsweise mit der Elektrode verbunden, beispielsweise mit dem Elektrodenblech verschweißt. Der drahtförmige Körper dient nicht unbedingt der Stromführung, sondern kann auch zu rein mechanischen Zwecken angeordnet sein, um das Verbiegen des Endabschnitts des Instruments und das Halten des Endabschnittes in der gewünschten verbogenen Form zu erlauben. Der drahtförmige Körper kann demnach der Leitung elektrischer Leistung in die Elektrode und/oder der mechanischen Stabilisierung der Elektrode dienen. Um mit dem Präparationsinstrument besonders präzise und isoliert präparieren zu können, ist eine dünne Elektrode wünschenswert. Mit abnehmender Dicke der Elektrode sinkt jedoch die mechanische Steifigkeit der Elektrode. Dies kann bei Bedarf durch den drahtförmigen Körper oder Abschnitt kompensiert werden. Darüber hinaus kann durch den drahtförmigen Körper oder Abschnitt der Wärmeabtransport von der Spitze des Präparationsinstruments durch den Endabschnitt des Präparationsinstruments verbessert werden.

Wenn, wie in Ausführungsbeispielen des erfindungsgemäßen Instruments, ein HF-Spatel mit einem Fluidapplikator kombiniert ist (Hybridspatel) kann dadurch die thermische Schädigung der Strukturen weitgehend reduziert und die Sicht auf den OP-Situs verbessert werden. Das Instrument erlaubt es, ein Fluid, beispielsweise ein Gas, eine Flüssigkeit oder eine Mischung aus beiden, zur Gewebeverdrängung auf das Gewebe zu applizieren bzw. in das Gewebe zur Gewebeelevation einzutragen. Dadurch können einzelne Gewebestrukturen bzw. deren Grenzen deutlicher dargestellt werden. Außerdem können durch die Ausgabe von Fluid Körperflüssigkeiten, wie unter anderem Blut, Gewebereste, Nebel, Rauch und dgl. weggespült bzw. verdrängt werden.

Bei der Applikation eines Fluids (insbesondere einer Flüssigkeit) mit hoher Leistungsdichte, dringt es in das Gewebe ein und reichert sich in den bindegewebsartigen Grenzbereichen zwischen der Zielstruktur und den angrenzenden Strukturen an, so dass diese auseinandergedrängt werden und ein vergrößerter (Sicherheits-)Abstand für die Manipulation mit dem Instrument entsteht (mechanischer und thermischer Schutz).

Wird hingegen ein Fluidstrom (insbesondere ein Gas) mit relativ niedriger Intensität in einem gewissen Abstand auf das Gewebe gerichtet, so kommt es infolge der Kraftwirkung des strömenden Fluids zu Verdrängungseffekten (Deformation) am Gewebe. Diese können aufgrund unterschiedlicher mechanischer Eigenschaften, z.B. der Elastizität der einzelnen Gewebestrukturen unterschiedlich stark ausgeprägt sein. Dadurch können zum Beispiel steifere Strukturen wie Gefäße gegenüber dem umgebenden weicheren Gewebe, wie beispielsweise Fett, deutlicher abgegrenzt werden.

Durch die Applikation eines Aerosol-Sprays kann außerdem eine Kühlung des Operationsbereichs und somit ein zusätzlicher Schutz vor thermischer Schädigung durch HF-Einwirkung realisiert werden. Da ein Aerosol die Rauchbildung während der HF-Anwendung reduziert, kann die Sicht auf den OP-Situs deutlich verbessert werden.

Um den Anwender bei der Vermeidung einer schadhaften Wirkung des Fluids, insbesondere Aerosols, zu unterstützen und/oder um insbesondere Gas- oder Aerosoleintrag in das Gewebe, z.B. ein Blutgefäß, zu verhindern, kann der Ausgang des Kanals, insbesondere der Ausgang der Düse, vorzugsweise proximal von dem distalen Ende der Elektrode zurückversetzt sein. Insbesondere wird so ein Aufsetzen des Ausgangs des Kanals auf das Gewebe verhindert. In bevorzugten Ausführungsformen ist das distale Ende des Kanals, an welchem der Ausgang angeordnet ist, von dem distalen Ende der Elektrode von einschließlich 2 mm bis einschließlich 10 mm zurückversetzt. In Ausführungsformen kann das distale Ende des Kanals bzw. der Ausgang des Kanals von dem distalen Ende der Elektrode beispielsweise zwischen 2 und 4 Millimeter in proximaler Richtung zurückversetzt sein.

Die spatelförmige Elektrode weist eine Oberseite und eine Unterseite auf, welche an den Rändern der Elektrode über je eine Seitenfläche verbunden sind. Der distale Endabschnitt des Kanals liegt bevorzugt in einer Ebene, welche von der Längsachse der Elektrode oder der Längsachse des drahtförmigen Abschnitts oder Körpers oder der Längsachse des Abschnitts der Elektrode bzw. des Isolierkörpers, der in Strömungsrichtung des Fluids aus dem Ausgang des Kanals nach dem Ausgang des Kanals angeordnet ist, und einer Flächennormalen der Oberseite oder der Unterseite der Elektrode aufgespannt wird. Der Ausrichtungswinkel (Azimuth) zwischen dem distalen Endabschnitt des Kanals und der Längserstreckungsrichtung der Elektrode und/oder eines drahtförmigen Körpers und/oder der Längsachse des Abschnitts der Elektrode bzw. des Isolierkörpers, der in Strömungsrichtung des Fluids aus dem Ausgang des Kanals nach dem Ausgang des Kanals angeordnet ist, ist entsprechend besonders bevorzugt 0°. Alternativ kann der Azimuth 0°± 10° oder 0°± 5° oder aus einem anderen Winkelbereich sein.. Der Azimuth wird bevorzugt in einer Ebene gemessen in welcher die spatelförmige Elektrode liegt oder derart gebogen werden kann, dass sie in dieser Ebene liegt, und/oder in welcher ein Abschnitt des Isolierkörpers oder der Elektrode liegt, welcher zwischen dem Ausgang des Kanals und dem distalen Ende des Isolierkörpers und/oder der Elektrode angeordnet ist.

Die Elektrode weist in Ausführungsformen des Instruments keine Öffnung auf, durch welche Gas, Flüssigkeit und/oder Aerosol hindurchgeleitet wird. Die Elektrode weist insbesondere vorzugsweise keinen in der Elektrode verlaufenden Kanal auf, der im Betrieb des Instruments ein Gas, ein Aerosol oder eine Flüssigkeit leiten würde.

Die Elektrode des erfindungsgemäßen Instruments schaut distal bevorzugt aus dem Isolierkörper heraus. Auf diese Weise geht von der Elektrode auch seitlich über den nicht isolierten Abschnitt eine Übertragung von HF-Leistung in das Gewebe aus.

Der Bereich der Oberseite und/oder der Bereich der Unterseite der Elektrode, der von dem Isolierkörper vorzugsweise freigestellt ist, ist vorzugsweise bogenförmig oder umgibt den Isolierkörper (in der Draufsicht) bogenförmig. Der freigestellte Bereich kann neben einem Abschnitt des Isolierkörpers enden, der zwischen dem Ausgang des Kanals und dem distalen Ende des Instruments angeordnet ist. Der freigestellte Bereich endet damit aus Sicht des Anwenders distal des Ausgangs. Alternativ kann der freigestellte Bereich neben einem Abschnitt des Isolierkörpers enden, der den Kanal enthält. Der freigestellte Bereich endet damit proximal des Ausgangs. Wenn der freigestellte Bereich proximal des Ausgangs des Kanals endet, erstreckt sich der freigestellte Bereich der Elektrode in Richtung proximal neben dem Ausgang des Kanals über den Ausgang des Kanals hinaus.

Die Freistellung durch den Isolierkörper kann auf solche Bereiche der Elektrode beschränkt sein, deren nächster Randflächenabschnitt eine Orientierung mit einer Komponente in distaler Richtung aufweist. Die Freistellung durch den Isolierkörper kann insbesondere auf solche Bereiche der Elektrode beschränkt sein, deren nächster Randflächenabschnitt ein Abschnitt der Stirnfläche der Elektrode ist.

Der Isolierkörper kann ein oder mehrteilig sein. Teile des Isolierkörpers können miteinander verbunden sein oder die Teile sind separat, voneinander beabstandet. Bevorzugt ist der Isolierkörper nahtlos einstückig ausgebildet. Die Elektrode kann in einer Ausnehmung in dem Isolierkörper angeordnet sein oder die Elektrode kann beispielsweise zwischen zwei nicht verbundenen Hälften des Isolierkörpers angeordnet sein, welche Abschnitte der Oberseite und der Unterseite der Elektrode flächig bedecken können.

Der Isolierkörper besteht vorzugsweise aus einem Polymer, insbesondere Kunststoff, beispielsweise Silikon. Bevorzugt ist der Isolierkörper flexibel um ein Verbiegen des distalen Endabschnitts des Instruments zuzulassen. Der Isolierkörper bedeckt Oberflächenabschnitte der Elektrode, um zu verhindern, dass das Gewebe von den bedeckten Oberflächenabschnitten aus mit chirurgisch wirksamer oder schädigender elektrischer Leistung beaufschlagt wird. Beispielsweise kann so ein unerwünschtes Austrocknen von Gewebeabschnitten verhindert werden. Von von dem Isolierkörper unbedeckten Oberflächenabschnitten aus kann das Gewebe durch die Oberflächenabschnitte mit chirurgisch wirksamer elektrischer Leistung beaufschlagt werden.

In bevorzugten Ausführungsformen wird der Isolierkörper durch Umspritzen der Elektrode hergestellt. Vorzugsweise wird dazu bei der Herstellung des erfindungsgemäßen Instruments in einer äußeren Form neben der Elektrode und/oder neben dem Elektrodenschaft, bevorzugt mit Abstand entlang der Elektrode und/oder dem Elektrodenschaft, ein Kern angeordnet, der den Kanal beim Umspritzen der Elektrode zumindest abschnittsweise freilässt oder bildet. Der Kern wird danach entfernt. Zurück bleibt der Kanal innerhalb der Wandstärke (Wanddicke) des die Elektrode umgebenden Isolierkörpers.

Die Elektrode weist vorzugsweise Ausnehmungen, insbesondere Löcher, zum Befestigen des Isolierkörpers an der Elektrode auf. Wenn der Isolierkörper durch Gießen, insbesondere Spritzgießen hergestellt wird, kann das Gussmaterial durch die Löcher treten und in den Löchern fest werden, so dass ein Formschluss zwischen dem Isolierkörper und der Elektrode hergestellt wird.

Die Elektrode ist vorzugsweise in einem Elektrodenschaft gefasst, wobei der Isolierkörper zumindest einen distalen Endabschnitt des Elektrodenschafts umgeben kann, um diesen elektrisch zu isolieren. Der drahtförmige Körper kann in oder an dem Elektrodenschaft befestigt sein, insbesondere in dem Elektrodenschaft stecken.

Der Kanal verläuft neben dem Elektrodenschaft oder beispielsweise abschnittsweise konzentrisch mit dem Elektrodenschaft.

Das Instrument kann einen Handgriff aufweisen, in welchem der Elektrodenschaft drehbar gelagert ist und der Anwender die Winkelstellung der Elektrode bezüglich des Handgriffs anpassen kann. Wenn der Kanal konzentrisch mit dem Elektrodenschaft verläuft, kann der Kanal zentral mit einer Gas-, Flüssigkeits- und/oder Aerosolleitung zur Gas-, Flüssigkeits- und/oder Aerosolquelle verbunden sein.

Es wird zudem eine Vorrichtung angegeben, welche ein erfindungsgemäßes Instrument und eine Gas-, Fluid- und/oder Aerosolquelle aufweist, wobei der Kanal des Instruments mit der Gas-, Fluid- und/oder Aerosolquelle verbunden ist, um den Kanal mit Gas, Flüssigkeit und/oder Aerosol zu speisen.

Weitere bevorzugte Merkmale und Ausführungsformen des erfindungsgemäßen Instruments und der erfindungsgemäßen Vorrichtung ergeben sich aus nachfolgender Beschreibung, den Figuren sowie den Unteransprüchen. Die Figuren zeigen:
Figur 1 - ein erfindungsgemäßes Instrument gemäß einer beispielhaften Ausführungsform in einer perspektivischen Ansicht,
Figur 2 - eine Draufsicht auf das distale Ende des Instruments gemäß Figur 1,
Figur 3 - das Instrument gemäß Figur 1 in perspektivischer Ansicht mit durch gestrichelte Linien angedeuteter Lage der Elektrode und des Kanals,
Figur 4 - eine beispielhafte Elektrodenbaugruppe mit Elektrodenschaft und Elektrode des Instruments gemäß Figur 1,
Figur 5 - eine ausschnittsweise Längsschnittansicht des Instruments gemäß Figur 1 entlang der Längsachse der Elektrode,
Figur 6a, 6b - Draufsichten auf Ausführungsbeispiele distaler Spatelabschnitte,
Figur 7a - eine perspektivische Ansicht auf den distalen Endabschnitt eines Beispiels einer weiteren Ausführungsform des erfindungsgemäßen Instruments,
Figur 7b - ein Längsschnittdarstellung des Beispiels gemäß Figur 7a,
Figur 8a - eine perspektivische Darstellung eines Beispiels einer dritten Ausführungsform des erfindungsgemäßen Instruments,
Figur 8b - eine Draufsicht auf eine Oberseite eines weiteren Beispiels gemäß einer dritten Ausführungsform des erfindungsgemäßen Instruments,
Figur 8c - eine perspektivische Darstellung eines noch weiteren Beispiels gemäß der dritten Ausführungsform des erfindungsgemäßen Instruments.

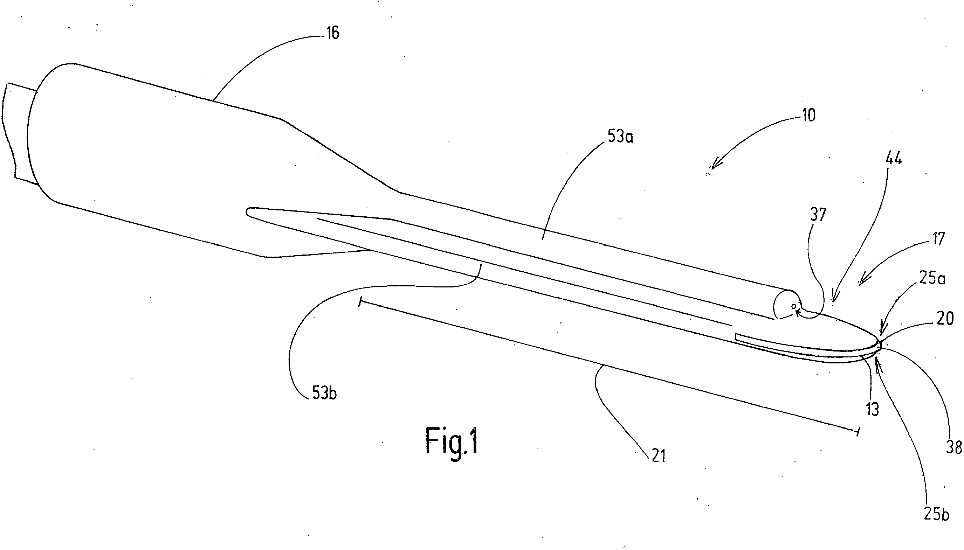

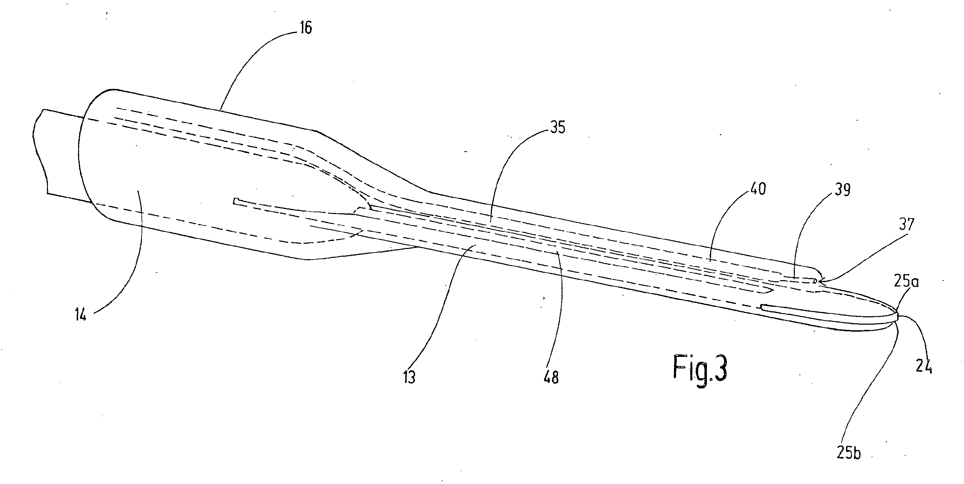

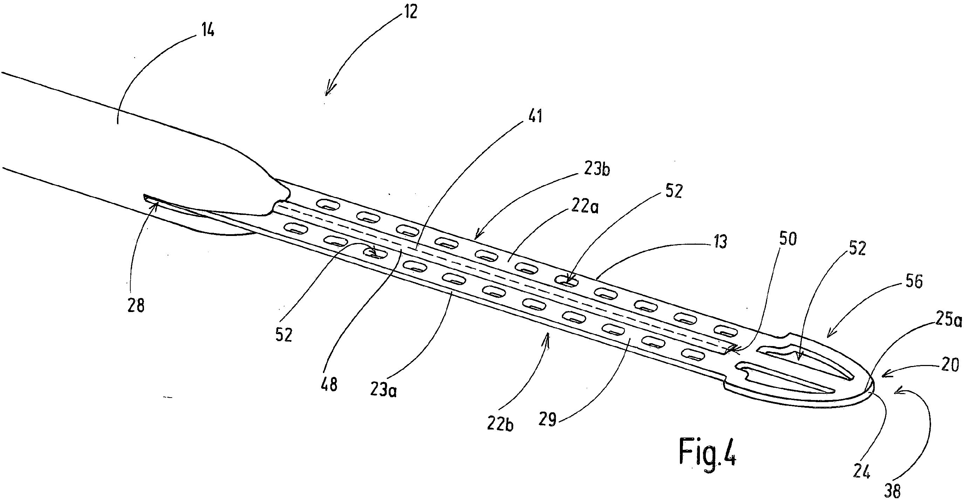

Einzelheiten eines Beispiels eines bevorzugten Aufbaus eines erfindungsgemäßen Präparationsinstruments 10 gemäß einer Ausführungsform sind in den Figuren 1 bis 5 veranschaulicht. Das Präperationsinstrument 10 weist eine Elektrodenbaugruppe 12 (s. insbesondere Figuren 3 und 4) mit einer Elektrode 13 und einem Elektrodenschaft 14 auf, wobei die Elektrode 13 in dem Elektrodenschaft 14 gehalten ist. Das proximale Ende des Elektrodenschaftes 14 kann lösbar in einem Handgriff (nicht gezeigt) des Instruments 10 stecken. Die Elektrodenbaugruppe 12 kann in dem Handgriff drehbar gelagert sein, um die Drehorientierung der Elektrodenbaugruppe 12 um die Längsachse des Elektrodenschaftes anzupassen. Die Elektrode 13 ist über den Elektrodenschaft 14 elektrisch mit einem Hochfrequenz-Generator (nicht gezeigt) verbunden. Die Elektrode 13 ist von einem Isolierkörper 16 umgeben, welcher die Elektrode 13 teilweise isoliert. Dies bedeutet, dass der Isolierkörper 16 die isolierten Abschnitten der Elektrode 13 bedeckt, um zu verhindern, dass das Gewebe quer durch die Oberfläche der isolierten Abschnitte mit chirurgisch wirksamer - insbesondere schneidender - oder schädigender elektrischer Leistung beaufschlagt wird. Der Isolierkörper 16 bedeckt Oberflächenabschnitte der Elektrode 13 vorzugsweise flächig oder schmiegt sich bevorzugt flächig an die Oberflächenabschnitte an, um diese zu isolieren. Der Isolierkörper 16 umgibt bevorzugt zumindest das Ende des Elektrodenschaftes 14 und erstreckt sich von dort bis zur Arbeitsspitze 17 des Instruments 10, wo der Isolierkörper 16 einen Randbereich 20 der Elektrode 13 freilässt. Das Präparationsinstrument 10 weist einen Endabschnitt 21 auf, der sich von dem Elektrodenschaft 14 bis zu dem distalen Ende des Präparationsinstruments 10 erstreckt.

Die Elektrode 13 weist eine Spatelform mit einer Oberseite 22a und einer in entgegengesetzte Richtung weisenden Unterseite 22b auf. Die Oberseite 22a und die Unterseite 22b sind miteinander mit je einer Seitenfläche 23a,b und distal mit einer Stirnfläche 24 verbunden, die an die Seitenflächen 23a,b angrenzt. Die Seitenflächen 23a, b und die Stirnfläche 24 bilden eine Randfläche der Elektrode 13.

Die Stirnfläche 24 ist bevorzugt vollständig oder zumindest teilweise nicht-isoliert. Die Seitenflächen 23a,b sind bis zu der Stirnfläche 24 bevorzugt isoliert. Die Elektrode 13 schaut distal vorzugsweise aus dem Isolierkörper 16 hervor. Bevorzugt zeigt die Elektrode 13 angrenzend an die Stirnfläche 24 aus dem Isolierkörper 16 heraus.

Die Elektrode 13 ist an den Seitenflächen 23a,b und/oder an der Stirnfläche 24 vorzugsweise frei von zu den Seitenflächen 23a,b oder der Stirnfläche 24 offenen Ausnehmungen, welche z.B. eine Hakenform der Elektrode 13 bilden könnten.

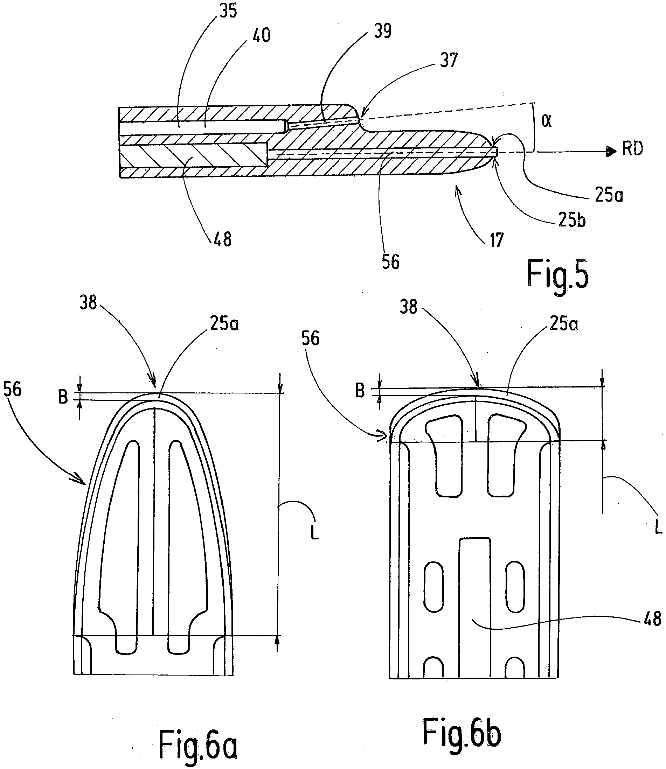

Besonders bevorzugt ist ein bogenförmiger (weißt die Form eines gekrümmten Streifens auf), an die Stirnfläche 24 angrenzender Abschnitt 25a der Oberseite 22a und/oder ein solcher Abschnitt 25b der Unterseite 22b nicht isoliert, wobei der oder die Abschnitte 25a,b in der Draufsicht auf Oberseite 22a und/oder Unterseite 22b, den Isolierkörper 16 umgeben. Der oder die streifenförmigen Abschnitte 25a,b grenzen an die Stirnfläche 24 an. Der oder die streifenförmigen Abschnitte 25a,b können beispielsweise eine Breite B von 0,05 Millimeter bis 2 Millimeter aufweisen. In den Ausführungsbeispielen gemäß Figur 6a und 6b weist der streifenförmige Abschnitt beispielsweise eine Breite B von 0,1 mm bis 0,15 mm auf.

Bevorzugt ist die Elektrode 13 von ihrem proximalen Ende 28 bis zu dem freigestellten streifenförmigen Abschnitt 25a,b geschlossen von dem Isolierkörper 16 umgeben. An den bis dorthin umgebenen Oberflächenabschnitt 29 der Elektrode 13 schließt ein Abschnitt der Stirnfläche 24 an, der um das distale Ende 38 der Elektrode 13 herum bis zurück zu dem umgebenen Oberflächenabschnitt 29 durchgängig freigestellt ist.

Die Breite des Endabschnitts 21 des Präparationsinstruments 10 wird vorzugsweise von der Breite 32 des Isolierkörpers 16 bestimmt. Die Elektrode 13 schließt bevorzugt mit dem Isolierkörper 16 ab oder weist eine geringere Breite 33 auf als der Isolierkörper 16.

Innerhalb des Isolierkörpers 16, in der Wand, welche die Elektrode 13 umgibt, verläuft ein Kanal 35 entlang der Elektrode 13. Der Kanal 35 und die Elektrode 13 erstrecken sich bevorzugt nebeneinander mit (wie dargestellt) oder ohne Abstand zwischen Kanal 35 und Elektrode 13. An dem distalen Ende 36 des Endabschnitts 21 weist der Kanal 35 einen Ausgang 37 auf. Der Ausgang 37 des Kanals 35 ist von dem distalen Ende 38 der Elektrode 13 vorzugsweise proximal zurückversetzt angeordnet. Der Ausgang 37 kann beispielsweise, parallel zur Elektrode 13 gemessen, zwischen wenigstens 2 Millimeter bis einschließlich 10 Millimeter zurückversetzt sein, beispielsweise zwischen einschließlich 2 Millimeter bis einschließlich 4 Millimeter.

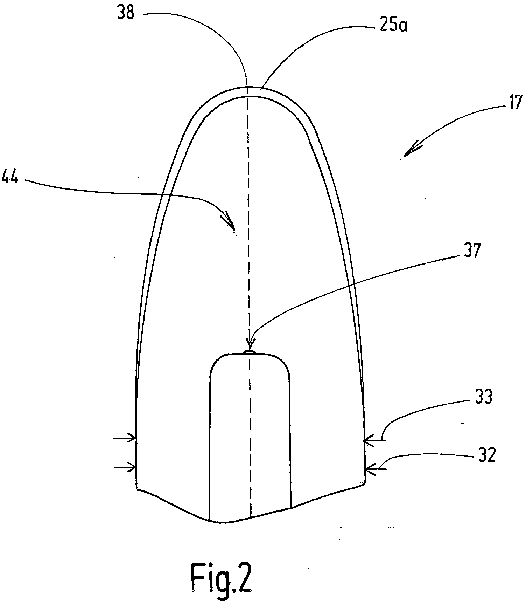

Der Kanal 35 ist in dem Isolierkörper 16 gemäß dargestellter Ausführungsform benachbart zu der Oberseite 22a oder beispielsweise benachbart der Unterseite 22b (nicht dargestellt) in dem Isolierkörper 16 angeordnet. Es kann auch je ein Kanal oder je ein Zweig eines Kanals benachbart zu der Oberseite 22a und benachbart zu der Unterseite 22b angeordnet sein (nicht dargestellt). Benachbart zu der anderen Seite, der Unterseite 22b oder der Oberseite 22a, kann kein Kanal angeordnet sein. Der Kanal 35 erstreckt sich von parallel (wie in Figur 3 veranschaulicht) oder zumindest abschnittsweise konzentrisch (nicht gezeigt) zu dem Elektrodenschaft 14 zunächst entlang der Elektrode 13 (bei gerader Elektrode 13 parallel zur Elektrode 13), wobei ein, bevorzugt gerader, Endabschnitt 39 des Kanals 35 relativ zu der Elektrode 13 bevorzugt angewinkelt ist, so dass sich der Winkel α, den die Längsachse des Endabschnitts 39 des Kanals 35 mit der Längsachse 41 der Elektrode 13 einschließt, in distale Richtung RD öffnet. Der Winkel α ist in Figur 5 dargestellt, welche eine Längsschnittansicht durch den distalen Endabschnitt des Instruments 10 veranschaulicht. Die entsprechende Schnittebene schneidet die Papierebene in Figur 2 senkrecht entlang der in Figur 2 gestrichelt eingezeichneten Schnittlinie. Der distale Endabschnitt 39 des Kanals 35 ist bezüglich der Längsachse 41 des Abschnitts der Elektrode 13 bzw. des Isolierkörpers 16, der in Strömungsrichtung des Fluids aus dem Ausgang 37 nach dem Ausgang 37 angeordnet ist, vorzugsweise angewinkelt mit einem Winkel α von größer 0° und bis 45°, besonders bevorzugt größer 5° bis kleiner 25°, beispielsweise 15±3°. Wenn der Kanal 35 mit Flüssigkeit oder Aerosol gespeist wird, tritt dieses aus dem Ausgang 37 des Kanals beispielsweise als Kegel, beispielsweise als Vollkegel aus. Der Kegel kann beispielsweise einen Öffnungswinkel (Kegelwinkel) von etwa 20° aufweisen. Auf Grund der Ausrichtung des distalen Endabschnitts 39 des Kanals 35 kann der Kegel derart ausgerichtet sein, dass der Kegel den Abschnitt des Isolierkörpers 16 bzw. der Elektrode 13 zwischen dem Ausgang 37 des Kanals 36 und dem distalen Ende 38 der Elektrode 13 bzw. des Instruments 10 nicht streift. In Ausführungsbeispielen kann der Fluidkegel derart ausgerichtet sein, dass der Sprühkegel"mantel" etwa parallel oder tangential zur Oberfläche des Abschnitts 44 des Instruments 10 ist, der sich von dem Ausgang 37 des Kanals 35 bis zum distalen Ende des Instruments 10 erstreckt. Würde der Strahlkegel den Abschnitt 44 hingegen treffen, kann dies zu einer erheblichen, unkontrollierten Tropfenbildung (Ansammlung von Flüssigkeit an der Elektrodenspitze) führen und könnte zum einen die elektrischen Eigenschaften des Spatels negativ beeinflussen, denn durch die Ansammlung von leitfähiger Flüssigkeit an der Spitze des Instruments kann die effektive Kontaktfläche der Elektrode vergrößert sein und die Ansammlung kann somit dem Ziel einer präzisen (isolierten) Präparation entgegenwirken, und zum anderen die Sicht auf das Operationsfeld einschränken.

Wie in den Figuren 3 und insbesondere 5 gezeigt, ist der distale Endabschnitt 39 des Kanals 35 vorzugsweise parallel entlang einer Ebene ausgerichtet oder liegt in der Ebene, welche senkrecht zu der Oberseite 22a oder Unterseite 22b der Elektrode steht und welche sich in Längserstreckungsrichtung der Elektrode 13, des drahtförmigen Abschnitts oder Körpers 48 oder in Längserstreckungsrichtung des Abschnitts der Elektrode 13 bzw. des Isolierkörpers 16, der in Strömungsrichtung des Fluids aus dem Ausgang 37 nach dem Ausgang 37 angeordnet ist, erstreckt. Der Azimuth zwischen dem distalen Endabschnitt 39 des Kanals 35 und der Längserstreckungsrichtung der Elektrode 13 und/oder des drahtförmigen Abschnitts oder Körpers 48 und/oder der Längsachse des Abschnitts der Elektrode 13 bzw. des Isolierkörpers 16, der in Strömungsrichtung des Fluids aus dem Ausgang 37 nach dem Ausgang 37 angeordnet ist, ist bevorzugt 0°, wobei der Azimuth zwischen der senkrechten Projektion der Längsachse des distalen Endabschnitts 39 des Kanals 35 auf eine Ebene, in welcher die spatelförmige Elektrode 13 liegt oder derart gebogen werden kann, dass sie in dieser Ebene liegt, und/oder in welcher ein Abschnitt des Isolierkörpers 16 oder der Elektrode 13 liegt, welcher zwischen dem Ausgang 37 des Kanals 35 und dem distalen Ende des Isolierkörpers 16 und/oder der Elektrode 13 angeordnet ist, und der Längserstreckungsrichtung der Elektrode 13 und/oder eines drahtförmigen Körpers 48 oder Abschnitts und/oder der Längsachse des Abschnitts der Elektrode 13 bzw. des Isolierkörpers 16, der in Strömungsrichtung des Fluids aus dem Ausgang 37 nach dem Ausgang 37 angeordnet ist, gemessen wird.

Der distale Endabschnitt 39 des Kanals 35 ist vorzugsweise ein Düsenabschnitt mit einem Strömungsquerschnitt, der kleiner ist, als der Strömungsquerschnitt des proximal an den distalen Endabschnitt 39 angrenzenden Kanalabschnitts 40. Der proximal angrenzende Kanalabschnitt 40 hat vorzugsweise einen Durchmesser von 1 Millimeter oder weniger, z.B. etwa 0,6 Millimeter.

Der distale Endabschnitt 21 des Instruments 10 mit dem Isolierkörper 16 und der Elektrode 13 ist bevorzugt flexibel, mit der bloßen Hand biegbar und bleibt nach dem Biegen vorzugsweise in einer von dem Anwender gewünschten Form. Der Endabschnitt 21 des Instruments 10 lässt sich folglich, insbesondere von einer geraden Ausrichtung der Elektrode 13 und des Isolierkörpers 16, in Richtungen biegen, um so die Form des Endabschnitts 21 des Instruments 10 an die chirurgische Aufgabe anzupassen.

Für ein besonders präzises Ausrichten des Endabschnitts 21 des Instruments 10 durch Biegen mit der Hand ist der Kanal 35 vorzugsweise durch einen in dem Isolierkörper 16 freigelassenen Hohlraum ausgebildet. Der Hohlraum verläuft, wie dargestellt, neben und bevorzugt mit Abstand zu dem Raum in dem Isolierkörper 16, welcher Raum von der Elektrode 13 ausgefüllt ist. Entsprechend bildet der Isolierkörper 16 vorzugsweise die den Kanal 35 begrenzende Wand. Die Wand, die den Kanal 35 umgrenzt, ist vorzugsweise nahtlos einstückig mit dem Isolierkörper 16 ausgebildet. Alternativ oder zusätzlich ist der distale Endabschnitt 39, insbesondere Düsenabschnitt, des Kanals bevorzugt durch einen in dem Isolierkörper 16 freigelassenen Hohlraum gebildet.

Das Instrument 10 ist bevorzugt dazu eingerichtet, so dass die Ausrichtung des Düsenabschnitts 39 bezüglich des distalen Elektroden Abschnitts 38 und/oder bezüglich der Längsachse 41 des Abschnitts des Isolierkörpers 16 und/oder des Abschnitts der Elektrode 13, der in Strömungsrichtung des Fluids durch den Kanal 35 gesehen nach dem Ausgang 37 (distal des Ausgangs 37) angeordnet ist, beim Biegen erhalten bleiben kann. Dies wird durch Ausbildendes Düsenabschnitts 39 in dem Bereich des distalen Endabschnitts 21 des Instruments 10 gewährleistet, der durch den Anwender zum Biegen des distalen Endabschnitts 21 umfasst wird und in dem folglich kein Biegemoment auftritt. Infolgedessen tritt die Biegung des distalen Endabschnitts 21 des Instruments 10 stets proximal des Düsenabschnittes 39 auf. Insbesondere kann der Abstand zwischen dem Ausgang 37 und/oder dem Düsenabschnitt 39 und dem distalen Ende 38 der Elektrode so klein gewählt sein, dass beim Verbiegen des Endabschnitts 21 die Biegung des distalen Endabschnitts 21 des Instruments 10 sicher stets proximal des Düsenabschnittes 39 ausgebildet wird.

Längs der Elektrode 13 ist vorzugsweise ein drahtförmiger Körper 48, bevorzugt aus Metall, angeordnet. Die Elektrode 13 kann einen länglichen Schlitz oder Ausnehmung aufweisen, die sich beispielsweise in Längserstreckungsrichtung der Elektrode vom proximalen Ende 28 der Elektrode 13 bis vor das distale Ende 38 der Elektrode 13 erstrecken kann, wobei der drahtförmige Körper 48 in den Schlitz oder der Ausnehmung angeordnet ist und die Ausnehmung - wie dargestellt - ausfüllen kann. Alternativ kann die Elektrode 13 beispielsweise einen drahtförmigen Abschnitt aufweisen, der zentrisch in der Elektrode 13 - in der Spatelform - angeordnet ist. Der Draht oder drahtförmige Abschnitt dient der Stabilisierung des distalen Endabschnitts 21 des Instruments 10 nach dem Verbiegen in der gewünschten Ausrichtung, in welche der distale Endabschnitt 21 durch Verbiegen gebracht ist. Der Draht dient auch der Stabilisierung des distalen Endabschnitts 21 in der Ausgangskonfiguration vor dem Verbiegen, so dass eine mechanische Manipulation des Gewebes möglich ist, ohne die Dicke des Endabschnitts 21 der Elektrode 13 zu vergrößern. Letzteres würde nämlich zu einer Verschlechterung der elektrischen Eigenschaften des HF-Energieeintrags führen, denn mit einer dickeren Elektrode 13 ist die Präzision, mit welcher präpariert werden kann, geringer als mit einer dünneren Elektrode 13.

Der drahtförmige Abschnitt oder Körper 48 endet bevorzugt vor dem distalen Ende 38 der Elektrode 13, so dass ein Bereich zwischen dem distalen Ende 50 des drahtförmigen Körpers 48 und dem distalen Ende 38 der Elektrode 13 frei von dem drahtförmigen Abschnitt oder Körper 48 ist.

Die Elektrode 13 selbst und/oder der drahtförmige Körper oder Abschnitt 48 bilden oder enthalten vorzugsweise keinen Fluidkanal.

Die Elektrode 13 kann, mit oder ohne drahtförmigem Abschnitt, mittels eines Stanzprozesses, oder fotochemischen Ätzens oder Laserschneidens hergestellt sein.

Der Isolierkörper 16 kann insbesondere aus Polymer, insbesondere Kunststoff, beispielsweise aus Silikon bestehen. Der Isolierkörper 16 ist bevorzugt durch Umgießen - insbesondere Umspritzen gemäß einem Spritzgußverfahren - der Elektrode 13 und des Elektrodenschafts 14 mit Isolierkörpermaterial gebildet. Der Kanal 35 wird bevorzugt als ein Hohlraum in dem Isolierkörper 16 durch Abformen mittels eines Kerns erzeugt. Damit kann darauf verzichtet werden, ein oder mehrere Kapillarrohre oder Schläuche, die zu einer höheren mechanischen Steifigkeit führen würden, in dem Isolierkörper 16 anzuordnen, wobei die Kapillarohre oder Schläuche den Kanal umschließen. Dadurch wird der distale Endabschnitt 21 des Instruments 10 mit der Elektrode 13 und dem Endabschnitt 39 des Kanals 35 durch Biegen genau ausrichtbar, ohne dass sich beim Biegen die Ausrichtung des Fluidstrahls, der den Ausgang 37 des Kanals 35 verlässt, bezüglich der Ausrichtung des distalen Endes 38 der Elektrode 13 ändert. Der distale Endabschnitt 39, insbesondere Düsenabschnitt des Kanals 35, kann ebenfalls integral, d.h. als ein freigelassenes Volumen in dem Isolierkörper 16 ausgebildet sein. Dadurch kann auf ein zusätzliches Bauelement wie z.B. ein Düsenrohr verzichtet werden. Dies ist insbesondere für die Montage von Ausführungsformen erfindungsgemäßer Instrumente 10 von Vorteil. Beim Gießen oder Umspritzen wird der Isolierkörper 16 bevorzugt um die Elektrode 13 herum gebildet, so dass die Elektrode von dem Isolierkörper 16 umschlossen wird. Zwischen Isolierkörper 16 und der Elektrode 13, beispielsweise zwischen einem Silikonkörper als Isolierkörper 16 und der Elektrode 13, ist vorzugsweise keine Zwischenschicht vorhanden. Vorzugsweise ist zwischen Isolierkörper 16 und der Elektrode 13, beispielsweise zwischen einem Silikonkörper als Isolierkörper 16 und der Elektrode 13 keine haftvermittelnde Schicht oder ein sonstiger Haftvermittler angeordnet.

Die Elektrode 13, insbesondere die Abschnitte der Elektrode 13 längs neben dem Draht 48 oder Drahtabschnitt, weist bevorzugt Ausnehmungen 52, insbesondere Löcher 52, auf, welche Ausnehmungen 52 beim Herstellen des Isolierkörpers 16 mit Isolierkörpermaterial gefüllt werden, um einen Formschluss zwischen Isolierkörper 16 und Elektrode 13 herzustellen, so dass der Isolierkörper 16 auch beim Verbiegen der Elektrode 13 mit dem Isolierkörper 16 durch Formschluss verbunden bleibt und dadurch stets eine bestimmte Ausrichtung relativ zu der Elektrode 13 aufweist.

Der Längsabschnitt 53a des Isolierkörpers 16, der den Kanal 35 enthält, ist vorzugsweise schmaler als der benachbarte Längsabschnitt 53b des Isolierkörpers 16, der die Elektrode 13 enthält. Auch dies fördert die genaue Umbiegbarkeit des Isolierkörpers 16. Der Längsabschnitt 53b des Isolierkörpers, der die Elektrode 13 enthält, insbesondere der Bereich zwischen Ausgang 37 des Kanals 35 und distalem Ende des Instruments 10 bzw. der Elektrode 13, weist vorzugsweise, wie veranschaulicht, ebenfalls eine Spatelform auf.

Der Kanal 35 kann, wie in Figur 3 gezeigt neben dem Elektrodenschaft 14 verlaufen. In anderen Ausführungsformen kann der Kanal 35 innerhalb des Elektrodenschafts 14 konzentrisch angeordnet sein (nicht gezeigt). Dies ist besonders vorteilhaft, wenn die Drehbarkeit des distalen Endabschnitts 21 des Instruments 10 um die Längsachse des Elektrodenschafts 14 relativ zu dem Handgriff geschaffen werden soll, so dass der Anwender die Drehorientierung des distalen Endabschnitts 21 des Instruments 10 bezüglich des Handgriffs, wie der Anwender wünscht, anpassen kann.

Wie in Figur 6a anhand eines Beispiels veranschaulicht, geht die Kontur der Elektrode 13 in manchen Ausführungsformen von dem distalen Ende 38 der Elektrode 13 in die Seiten der Elektrode über, ohne nach vorne (distal) orientierte abgerundete Ecken zu bilden. In Figur 6b ist hingegen ein Beispiel einer Ausführungsform dargestellt, welches solche abgerundete Ecken 55a, b bildet.

Die laterale Kontur der Elektrodenspitze 56 kann sich zum distalen Ende 38 der Elektrode hin verjüngen (Spitzprofil, s. Figuren 2, 4, 6a), weitgehend parallel laufen (Rechteckprofil, s. Figur 6b) oder sich auch erweitern (Trapezprofil, nicht dargestellt). Dabei kann der freigestellte Abschnitt der Randfläche der Elektrode entweder umlaufend (über den gesamten Umfang des spatelförmigen distalen Endabschnitts 21 des Instruments 10) oder aber auf einen distalen Teil des vorzugsweise spatelförmigen distalen Endabschnitts 21 des Instruments 10 begrenzt ausgeführt sein.

Die von der Stirnfläche festgelegte Breite der Elektrode geht an dem Übergang zu den Seitenflächen und/oder an dem Übergang von dem freigestellten Bereich der Oberseite 22a und/oder der Unterseite 22b auf den nicht freigestellten Bereich vorzugsweise abrupt auf eine kleinere Breite über, die von dem Abstand der Seitenflächen 23a, b voneinander festgelegt ist. In Ausführungsformen mit Spitzprofil (vgl. Fig. 2, 4, 6a) kann der Übergang von dem distalen Ende 38 der Elektrode 13, in Längserstreckungsrichtung der Elektrode 13 gemessen, beispielsweise einen Abstand entsprechend wenigstens der Breite 32 des Isolierkörpers 16 im Endabschnitt 21 des Instruments 10 und/oder entsprechend wenigstens der größten Breite 33 der Elektrode 13 im distalen Endabschnitt 21 des Instruments 10 aufweisen.

In Ausführungsformen mit Rechteckprofil (vgl. Fig. 6b) kann der Übergang von dem distalen Ende 38 der Elektrode 13 in Längserstreckungsrichtung der Elektrode 38 gemessen, beispielsweise einen Abstand von höchstens einem Drittel der Breite 32 des Isolierkörpers 16 im Endabschnitt des Instruments 10 und/oder der größten Breite 33 der Elektrode 13 im distalen Endabschnitt 21 des Instruments 10 aufweisen.

Die Länge L des freigestellten Bereichs der Elektrode 13 kann beispielsweise bis zu dem zweifachen der Breite 32 des Endabschnitts 21 des Instruments 10 betragen, wobei die Länge L in Längserstreckungsrichtung der Elektrode 13 gemessen wird. Die Länge des freigestellten Bereichs kann z.B. 4,5 mm oder weniger betragen.

Der Kanal 35 ist vorzugsweise mit einer Pumpe oder einer Druckquelle verbunden, um den Kanal mit Gas, Flüssigkeit und/oder Aerosol zu speisen.

Das erfindungsgemäße Instrument 10 wird beispielsweise wie folgt hergestellt:
In einer äußeren Form (nicht dargestellt) wird eine Elektrodenbaugruppe 12 mit einem Elektrodenschaft 14 und einer Elektrode 13 bereitgestellt und neben der Elektrode 12 und dem Elektrodenschaft 14, bevorzugt mit Abstand zu der Elektrode 12 und/oder dem Elektrodenschaft 14, wird eine innere längliche Form (länglicher Kern) (nicht dargestellt) angeordnet. Es wird Isolierkörpermaterial in die äußere Form gespritzt, so dass dieses den Isolierkörper 16 um den Elektrodenschaft 14 und die Elektrode 13 bildet. Der längliche Kern sorgt dafür, dass in dem Isolierkörper 16 der Kanal 35 freigelassen wird.

Das erfindungsgemäße Instrument arbeitet beispielsweise wie folgt:
Das erfindungsgemäße Instrument 10 kann beispielsweise in der offenen oder der laparoskopischen oder der endoskopischen Chirurgie eingesetzt werden. Zur HF-chirurgischen Behandlung, insbesondere Präparation, des Gewebes wird die Elektrode 13 mit HF-Leistung beaufschlagt und kann dann als Schneidwerkzeug eingesetzt werden, um Gewebe zu schneiden. Die Ausgabe eines Fluids, insbesondere Aerosolstrahls, aus dem Ausgang 37 des Kanals 35 kann beispielsweise automatisch mit der Aktivierung der Beaufschlagung der Elektrode 13 mit HF-Leistung aktiviert werden. In Ausführungsformen kann die Ausgabe eines Fluid-, insbesondere Aerosolstrahls, beispielsweise unabhängig von der Aktivierung der HF-Leistungsabgabe durch die Elektrode 13 aktivierbar sein.

Mit dem erfindungsgemäßen Präparationsinstrument 10, in welchem ein HF-Spatel (spatelförmige Elektrode 13) mit einem Fluidapplikator 38 (Pumpe oder Druckquelle und Kanal), insbesondere Aerosolapplikator, kombiniert ist (Hybridspatel) kann durch die Ausgabe eines Fluid, insbesondere Flüssigkeits- oder Aerosolstrahls, die Sicht auf den OP-Situs verbessert und damit präziser und sicherer gearbeitet werden. Mit dem erfindungsgemäßen Instrument 10 lässt sich Gewebe schonend und präzise präparieren. Das erfindungsgemäße Instrument 10 unterstützt den Anwender darin, eine unbeabsichtigte thermische Schädigung sensibler (zu präparierender) oder angrenzender Strukturen zu vermeiden, da die Ausgabe des Fluidstrahls, insbesondere Flüssigkeits- und Aerosolstrahls, die Vorbereitung durch Reinigung oder Präparation des OP-Situs erlaubt, so dass die Kontaktfläche zwischen dem Gewebe und der HF-Elektrode 13 dem Anwender zu jedem Zeitpunkt bekannt bzw. durch ihn kontrollierbar ist und zudem die einzelnen Gewebegrenzen für den das Instrument 10 anwendenden Chirurgen stets deutlich erkennbar sind. Das Instrument 10 ist bevorzugt dazu eingerichtet, ein Gas, eine Flüssigkeit oder eine Mischung aus beiden (Aerosol) auf das Gewebe zu applizieren (Gewebeverdrängung), um unter anderem Blut wegzuspülen und einzelne Gewebestrukturen bzw. deren Grenzen deutlicher darzustellen. Alternativ oder zusätzlich kann das Instrument 10 dazu eingerichtet sein, Flüssigkeit oder Aerosol in das Gewebe einzubringen, um eine Gewebeschicht anzuheben (Gewebeelevation).

Bei der Applikation eines Fluids (insbesondere einer Flüssigkeit) mit hoher Leistungsdichte, dringt das Fluid in das Gewebe ein und reichert sich in den bindegewebsartigen Grenzbereichen zwischen der Zielstruktur und den angrenzenden Strukturen an, so dass diese auseinandergedrängt werden und ein vergrößerter (Sicherheits-)Abstand für die Manipulation mit dem Instrument 10 entsteht (mechanischer und thermischer Schutz).

Wird mit einem erfindungsgemäßen Instrument 10 hingegen ein Fluidstrom (insbesondere ein Gas oder ein Aerosol) mit relativ niedriger Intensität in einem gewissen Abstand auf das Gewebe gerichtet, so kommt es infolge der Kraftwirkung des strömenden Fluids zu Verdrängungseffekten (Deformation) am Gewebe. Diese können aufgrund unterschiedlicher mechanischer Eigenschaften, z.B. der Elastizität der einzelnen Gewebestrukturen unterschiedlich stark ausgeprägt sein. Dadurch können zum Beispiel steifere Strukturen, wie Gefäße, gegenüber dem umgebenden weicheren Gewebe, wie z.B. Fett, deutlicher abgegrenzt werden.

Durch die Applikation eines Aerosol-Sprays kann außerdem eine Kühlung des Operationsbereichs und somit ein zusätzlicher Schutz vor thermischer Schädigung durch HF-Einwirkung realisiert werden.

Schließlich kann das mit Hilfe des Kanals 35 des erfindungsgemäßen Instruments ausgebrachtem Aerosol die Rauchbildung während der HF-Anwendung reduziert werden, um dadurch eine deutlich verbesserte Sicht auf den OP-Situs zu erhalten.

Mit einem proximal von dem distalen Ende 38 der Elektrode 13 zurückgesetzten Ausgang 37 des Kanals 35 ist die Gefahr einer Schädigung von Gewebe mit dem Aerosol vermieden und/oder ein Aerosoleintrag in das Gewebe, z.B. ein Blutgefäß, verhindert. Insbesondere wird so ein Aufsetzen des Ausgangs 37 des Kanals 35 auf das Gewebe verhindert.

Mit dem erfindungsgemäßen Instrument 10 wird folglich ein Bedarf an einem Präparationsinstrument gedeckt, das ein präzises und schonendes Freilegen/Darstellen sensibler Gewebestrukturen erlaubt.

Bevorzugt kann, wie oben beschrieben, der Anwender den Endabschnitt 21 des Instruments 10 mit der Hand biegen, so dass die Ausrichtung des Düsenabschnitts 39 bezüglich des distalen Elektroden Abschnitts 38 und/oder bezüglich der Längsachse 41 des Abschnitts des Isolierkörpers 16 und/oder der Längsachse 41 des Abschnitts der Elektrode 13, der in Strömungsrichtung des Fluids durch den Kanal 35 gesehen nach dem Ausgang 37 (distal dem Ausgang 37) angeordnet ist, erhalten bleibt.

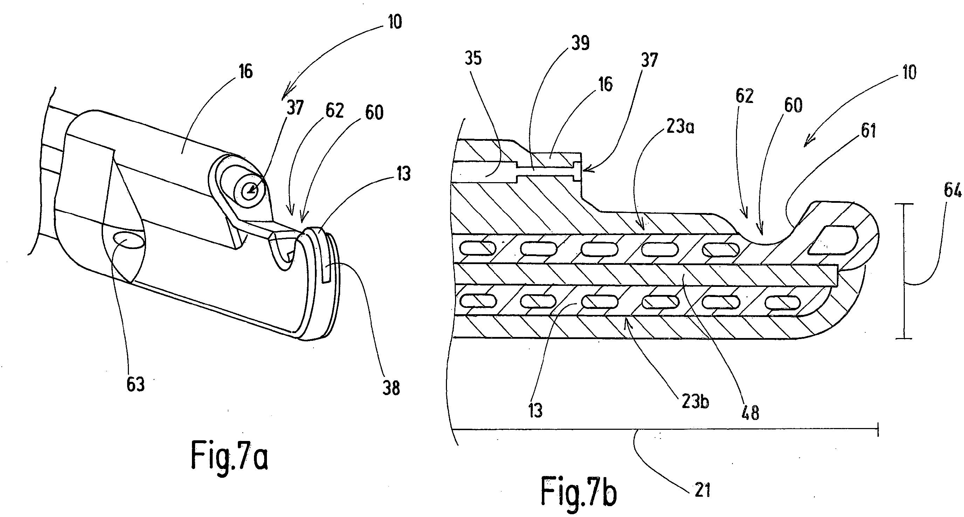

Figur 7a und Figur 7b zeigen ein Beispiel einer weiteren Ausführungsform des erfindungsgemäßen Instruments 10. Diese Ausführungsform weist eine spatelförmige Elektrode 13 auf, die eine Längsausnehmung aufweisen kann, in welcher ein drahtförmiger Körper 48 angeordnet ist, oder welche beispielsweise einen zentralen drahtförmigen Abschnitt aufweist. Im Unterschied zu der Ausführungsform gemäß Figuren 1 bis 5 ist der Kanal 35 in dem Isolierkörper 16 benachbart zu einer schmalen Seitenfläche 23a der Elektrode 13 und nicht, wie in den Ausführungsbeispielen gemäß Figuren 1 bis 5, benachbart zu der breiteren Oberseite 22a der Elektrode 13 angeordnet. Der Endabschnitt 39 des Kanals 35 kann - anders als dargestellt, in einem Winkel *α* relativ zu der Längserstreckungsrichtung des distalen Endabschnitts 21 bzw. der Elektrode 13 angeordnet sein, so dass sich der Winkel in distale Richtung öffnet. In dem dargestellten Ausführungsbeispiel verläuft der Endabschnitt 39 des Kanals 35 parallel zu der Längserstreckungsrichtung der Elektrode 13 bzw. zur Längserstreckungsrichtung des distalen Endabschnitts 21. Die Elektrode 13 kann an einer oder beiden Seitenflächen 23a, b eine Hakenausnehmung 60 aufweisen. Der von dem Isolierkörper 16 freigestellte Bereich 61 der Elektrode 13 innerhalb der Hakenausnehmung 62 des Isolierkörpers 16 kann von dem Gas-,Aerosol- oder Flüssigkeitsstrahl getroffen werden, um diesen zu reinigen. Alternativ oder zusätzlich kann Gewebe, welches in dem Bereich innerhalb der hakenförmigen Ausnehmung 60, 62 behandelt werden soll, zuvor oder gleichzeitig mit einem Strahl aus dem Kanal 35 beaufschlagt werden. Alternativ oder zusätzlich können durch Beaufschlagen mit einem Gas-,Aerosol- oder Flüssigkeitsstrahl, an dem Gewebe, das mit dem distalen Ende der Elektrode 38 in Kontakt ist, Verdrängungseffekte (Deformation) erzeugt werden. Dadurch können einzelne Strukturen gegeneinander deutlicher abgegrenzt werden. In dem Isolierkörper 16 kann ein Absaugkanal 63 angeordnet sein. Wie auch der Kanal 35 zum Ausgeben eines Gases-, Flüssigkeit-, Aerosolstrahles an oder in das Gewebe oder zwischen Gewebestrukturen kann der Isolierkörper 16 auch die Kanalwand des Absaugkanals 63 bilden. Die Elektrode 13 steht seitlich über den Isolierkörper 16 über, so dass die Breite 64 des Abschnitts des Instruments 10 zwischen dem Ausgang 37 des Kanals 35 und dem distalen Ende des Instruments 10 von der Elektrode 13 und dem Isolierkörper 16 bestimmt wird.

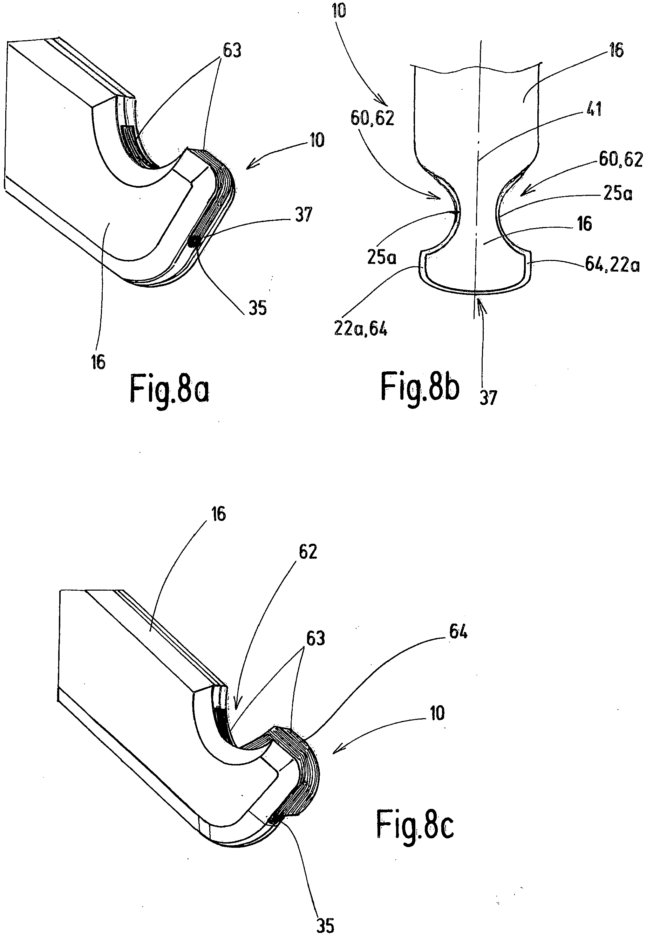

Die Figuren 8a und 8b zeigen Beispiele von Ausführungsformen in denen der Kanal 35 in der Elektrode 13 gebildet ist. Der in die Elektrode 13 integrierte Kanal 35 kann einen Ausgang 37 am distalen Ende des Instruments 10 aufweisen. Die Elektrode 13 kann sowohl distal als auch seitlich nicht über den Isolierkörper 16 überstehen (Figur 8a), sondern beispielsweise mit dem Isolierkörper 16 abschließen, so dass nur Seitenflächenabschnitt 63 von dem Isolierkörper 16 unbedeckt ist. Andernfalls kann die Elektrode 13 beispielsweise nicht distal über den Isolierkörper 16 überstehen, sondern nur seitliche Abschnitte 64 der Oberseite 22a und/oder der Unterseite 22b der Elektrode 13 können, wie veranschaulicht, von dem Isolierkörper 16 unbedeckt sein. Die dargestellten Ausführungsbeispiele unterscheiden sich außerdem darin, dass in Figur 8a nur eine Seitenfläche 23a des Instruments 10 einen Haken durch eine Ausnehmung 60, 62 in den Isolierkörper 16 von der Elektrode 13 aufweist und in Figur 8b beide Seitenflächen 23a, b einen Haken aufweisen.

Das Beispiel gemäß Figur 8c unterscheidet sich von dem Beispiel gemäß Figur 8a insbesondere dadurch, dass die Elektrode 13 in der Hakenausnehmung 62 des Isolierkörpers 16 als auch distal und darüber hinaus seitlich über die angrenzende Kontur des Isolierkörpers 16 übersteht.

In dem Isolierkörper kann eine Ausnehmung vorhanden sein (nicht dargestellt), durch welche ein flächiger Abschnitt der Elektrode zum Koagulieren freigestellt ist, um einen Koagulationsbereich zu bilden. Alternativ zu einer Freistellung kann ein Koagulationsbereich gebildet sein (nicht dargestellt), indem ein Abschnitt des Isolierkörpers eine Porosität aufweisen, welche derart angepasst ist, dass der Isolierkörper in diesem Abschnitt die Elektrode nicht isoliert, um eine Beaufschlagung des Gewebes mit elektrischer Leistung zur Koagulation des Gewebes durch diesen Abschnitt zuzulassen.

Es wird ein Präparationsinstrument 10 (Hybridinstrument) angegeben, welches ein HF-Instrument mit einer mittels eines Isolierkörpers 16 teilweise isolierten Elektrode 13 aufweist, welches mit einem Fluidapplikator mit einem in dem Isolierkörper 16 angeordneten Kanal 35 zur Applikation eines Fluids an oder in Gewebe kombiniert ist. In besonders bevorzugten Ausführungsformen des Präparationsinstruments 10 ist die Elektrode 13 eine Spatelelektrode, welche in dem Isolierkörper 16 steckt, welcher Abschnitte 24, 25a, 25b, 63, 64 der Oberfläche der Elektrode 13 nicht bedeckt, so dass diese Abschnitte 24, 25a, 25b, 63, 64 Gewebekontakt haben können. In den besonders bevorzugten Ausführungsformen bildet der Isolierkörper 10 vorzugsweise die den Kanal 35 begrenzende Kanalwand. In den besonders bevorzugten Ausführungsformen sind der Isolierkörper 16 und die Elektrode 13 flexibel, um die Form des Isolierkörpers 16 und der Elektrode 13 zusammen an die chirurgische Aufgabe anzupassen.

### Bezugszeichenliste:

| | |
|---|---|
| 10 | Präparationsinstrument/Instrument |
| 12 | Elektrodenbaugruppe |
| 13 | Elektrode |
| 14 | Elektrodenschaft |
| 16 | Isolierkörper |
| 17 | Arbeitsspitze |
| 20 | Randbereich der Elektrode |
| 21 | Endabschnitt |
| 22a | Oberseite |
| 22b | Unterseite |
| 23a,b | Seitenflächen |
| 24 | Stirnfläche |
| 25a,b | Abschnitte |
| 28 | Proximales Ende der Elektrode |
| 29 | Oberflächenabschnitt |
| 32 | Breite des Isolierkörpers |
| 33 | Breite der Elektrode |
| 35 | Kanal |
| 36 | Distales Ende |
| 37 | Ausgang des Kanals |
| 38 | Distales Ende der Elektrode |
| 39 | Endabschnitt des Kanals/Düsenabschnitt |
| 40 | Proximal angrenzender Abschnitt des Kanals |
| 41 | Längsachse der Elektrode |
| 44 | Abschnitt des Instruments |
| 48 | Drahtförmiger Körper |
| 50 | Distales Ende des drahtförmigen Körpers/Abschnitts |
| 52 | Ausnehmung |
| 53a,b | Längsabschnitte |
| 55a,b | Ecken |
| 56 | Elektrodenspitze |
| 60 | Hakenausnehmung |
| 61 | Bereich |
| 62 | Hakenausnehmung |
| 63 | Seitenflächenabschnitt |
| 64 | Abschnitt |
| α | Winkel |
| RD | Distale Richtung |
| L | Länge |
| B | Breite |

## Patentansprüche

1. HF-chirurgisches Präparationsinstrument (10) zum Einwirken auf Gewebe, wobei das Instrument (10) eine Elektrode (13) aufweist, welche mittels eines Isolierkörpers (16) teilweise elektrisch isoliert ist, wobei innerhalb des Isolierkörpers (16) ein Kanal (35) zum Ausbringen einer Flüssigkeit, eines Gases oder eines Aerosols angeordnet ist, wobei der Kanal (35) an eine Flüssigkeits-, Gas und/oder Aerosolquelle anschließbar ist.

2. Instrument (10) nach einem der vorstehenden Ansprüche, wobei der Isolierkörper (16) den Kanal (35) umfänglich begrenzt.

3. Instrument (10) nach einem der vorstehenden Ansprüche, wobei der Kanal einen Düsenabschnitt (39) aufweist, der von dem Isolierkörper (16) umfänglich begrenzt wird.

4. Instrument (10) nach einem der vorstehenden Ansprüche, wobei der distale Endabschnitt (21) des Instruments (10) biegbar ist, um die Form des distalen Endabschnitts (21) des Instruments (10) an die Behandlungsaufgabe anzupassen.

5. Instrument (10) nach einem der vorstehenden Ansprüche, wobei der Ausgang (37) des Kanals (35) proximal von dem distalen Ende (38) der Elektrode (13) und/oder von dem distalen Ende (38) des Instruments (10) zurückversetzt ist.

6. Instrument (10) nach einem der vorstehenden Ansprüche, wobei das der Ausgang (37) des Kanals (35) von dem distalen Ende (38) der Elektrode (13) und/oder von dem distalen Ende (38) des Instruments (10) von einschließlich 2 mm bis 10 mm zurückversetzt ist.

7. Instrument (10) nach einem der vorstehenden Ansprüche, wobei die Elektrode (13) distal aus dem Isolierkörper (16) heraus schaut.

8. Instrument (10) nach einem der vorstehenden Ansprüche, wobei die Elektrode (13) eine Spatelform hat.

9. Instrument (10) nach einem der vorstehenden Ansprüche, wobei die Elektrode (13) in der Spatelform (48) einen drahtförmigen Abschnitt (48) aufweist oder wobei die Elektrode (13) eine Ausnehmung aufweist, in welcher ein drahtförmiger Körper (48) angeordnet ist.

10. Instrument (10) nach einem der vorstehenden Ansprüche, wobei der Isolierkörper (16) durch Umspritzen der Elektrode (13) gebildet ist.

11. Instrument (10) nach einem der vorstehenden Ansprüche, wobei die Elektrode (13) Ausnehmungen (52) zum Befestigen des Isolierkörpers (16) an der Elektrode (13) aufweist.

12. Instrument (10) nach einem der vorstehenden Ansprüche, wobei die Elektrode (13) in einem Elektrodenschaft (14) gefasst ist, wobei der Isolierkörper (16) einen distalen Endabschnitt des Elektrodenschafts (14) umgibt.

13. Instrument (10) nach einem der vorstehenden Ansprüche, wobei der Kanal (35) neben dem Elektrodenschaft (14) oder abschnittsweise konzentrisch mit dem Elektrodenschaft (14) verläuft.

14. Instrument (10) nach einem der vorstehenden Ansprüche, wobei ein von dem Isolierkörper (16) freigestellter Bereich der Elektrode (13) den Isolierkörper (16) in der Draufsicht bogenförmig umgibt.

15. Vorrichtung mit einem Instrument (10) nach einem der vorstehenden Ansprüche und mit einer Fluidquelle und/oder einer Aerosolquelle, wobei der Kanal (35) des Instruments (10) mit der Fluidquelle und/oder der Aerosolquelle verbunden ist, um den Kanal (35) zu speisen.
